# EUROPEAN PATENT SPECIFICATION

(11) **EP 2 391 321 B1**
(45) Date of publication and mention of the grant of the patent: **15.03.2017**
(21) Application number: 09700023.6
(22) Date of filing: 30.01.2009
(51) Int. Cl.: A61F 11/06, G10K 11/178

(54) **SYSTEM AND METHOD FOR PROVIDING ACTIVE HEARING PROTECTION TO A USER**
SYSTEM UND VERFAHREN ZUR BEREITSTELLUNG EINES AKTIVEN GEHÖRSCHUTZES FÜR EINEN ANWENDER
SYSTÈME ET PROCÉDÉ PERMETTANT D'OFFRIR UNE PROTECTION AUDITIVE ACTIVE À UN UTILISATEUR

(43) Date of publication of application: 07.12.2011
(73) Proprietor: Sonova AG, 8712 Stäfa (CH)
(72) Inventor: MÜLDER, Hans, 3184 Wünnewil (CH); HARSCH, Samuel, 1338 Ballaigues (CH)
(74) Representative: Schwan Schorer & Partner mbB
(86) International application number: PCT/EP2009/051100
(87) International publication number: WO 2009/050306

(56) References cited:
- WO-A-2006/047600
- WO-A-2007/082579
- US-B1- 7 065 219

## Description

The invention relates to a hearing protection system wherein each of the left ear and right ear hearing protection devices comprises at least one microphone and a loudspeaker, i.e. an active unit, in order to provide for a defined acoustic attenuation of ambient sound. The invention also relates to a method for providing active hearing protection. In particular, the invention relates to so-called music filters (musicians' earplugs).

Musicians are often exposed to high sound pressure levels during play. Hearing loss among professional (and amateur) musicians is a well-known phenomenon. To protect the ears while maintaining a natural experience of music, music filters are available and widely used, both during rehearsals and performances. These filters are all passive filters with a (fairly) flat frequency characteristic, i.e. damping is more or less independent of frequency and sound pressure level. Conventional (passive) earplugs reduce sound more at high frequencies than at low and medium frequencies, which makes music and voices unclear and unnatural. Disadvantages of such conventional music filters are that non-linearities in sound perception are not taken into account and that damping of soft passages in music may be too strong. For example, musicians' earplugs commercially available under the designation "Etymotic ER25", which are recommended for brass, percussion and amplified instruments, reduce sound by approximately 25 dB, independent of the level of the sound.

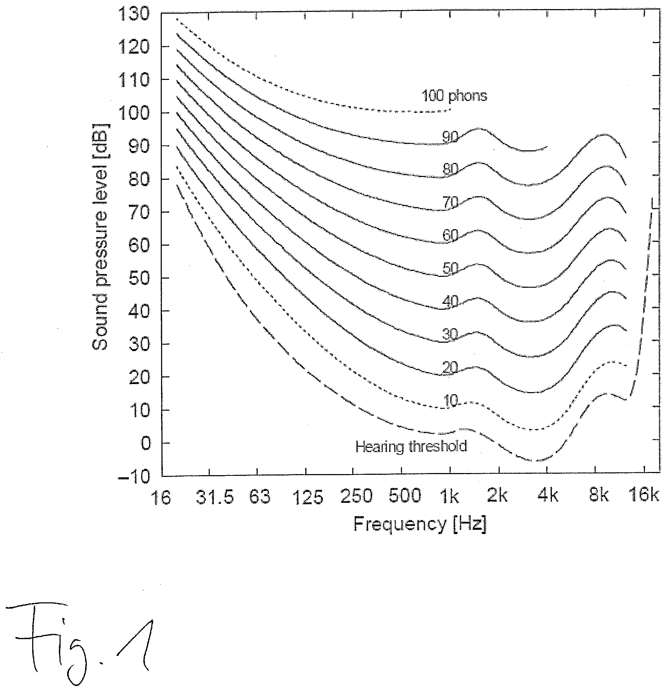

However, the curves of equal loudness (the so-called isophones) of the normal hearing ear are not equidistant with regard to frequencies and levels. This means that level- and frequency-independent damping will alter the loudness relationship between different frequencies, especially when low frequencies are present. In room acoustics this phenomenon is known as the Bass Loss Problem. In Fig. 1 an example of curves of equal loudness is shown, wherein the sound pressure level in dB required for achieving certain loudness perception is shown as a function of frequency for different loudnesses from 0 to 100 phons.

EP 1 674 061 A1 relates to an active hearing protection system, wherein one of several gain models is automatically selected according to the present auditory scene. At low noise levels, a transparent mode providing for low attenuation is selected, whereas at high noise levels an attenuation mode comprising high attenuation is selected.

WO 2007/082579 A2 relates to an active hearing protection system which comprises a communication function and which is connected to a wireless communication device.

DE 198 59 480 A1 relates to a hearing aid comprising a limiter for the audio signal output level, wherein the maximum output level is defined as a function of the frequency according to the isophones in order to achieve substantially the same loudness perception at the maximal level for all frequencies.

EP 1 207 718 A2 relates to a hearing aid, wherein isophones are taken into account for audio signal processing.

US 7,409,066 B2 relates to a car radio system, wherein isophones are taken into account in audio signal processing, in order to achieve a hearing impression as natural as possible.

It is an object of the invention to provide for a hearing protection system providing for a hearing impression as natural as possible, in particular for use by profesional and amateur musicians and music lovers. It is also an object of the invention to provide for a corresponding method for providing hearing protection to a user.

According to the invention, these objects are achieved by a hearing protection system as defined in claim 1 and a method as defined in claim 11, respectively.

The invention is beneficial in that, by dividing the input audio signals into a plurality of frequency bands and selecting the gain applied within each frequency band as a function of the frequency of the respective frequency band and the level of the input audio signals falling within the respective frequency band in such a manner that, by taking into account the shapes of the curves of equal loudness and the passive attenuation provided by the respective hearing protection device, the total attenuation provided by the respective hearing protection device in each of the frequency bands corresponds to the same target loudness difference, the non-linearities of sound perception are fully taken into account, so that a close to natural hearing impression is achieved, with the user only experiencing a reduction in loudness which is the same for all frequencies. This is particularly important for listening to music.

Preferably, the audio signal processing unit is designed to select for each frequency band the same gain for both hearing protection devices, wherein the gain for each frequency band is selected according to the higher one of the sound pressure levels of the input audio signals captured by each of the hearing protection devices. Thereby localization cues based on level differences between left and right ear can be preserved as far as possible.

Further preferred embodiments of the invention are defined in the dependent claims.

Examples of the invention will be illustrated by a reference to the attached drawings, wherein:
- Fig. 1: is an example of curves of equal loudness;
- Fig. 2: is a schematic view of a mechanical configuration of a hearing protection system according to the invention; and
- Fig. 3: is a block diagram of electronic components of a hearing protection system according to the invention.

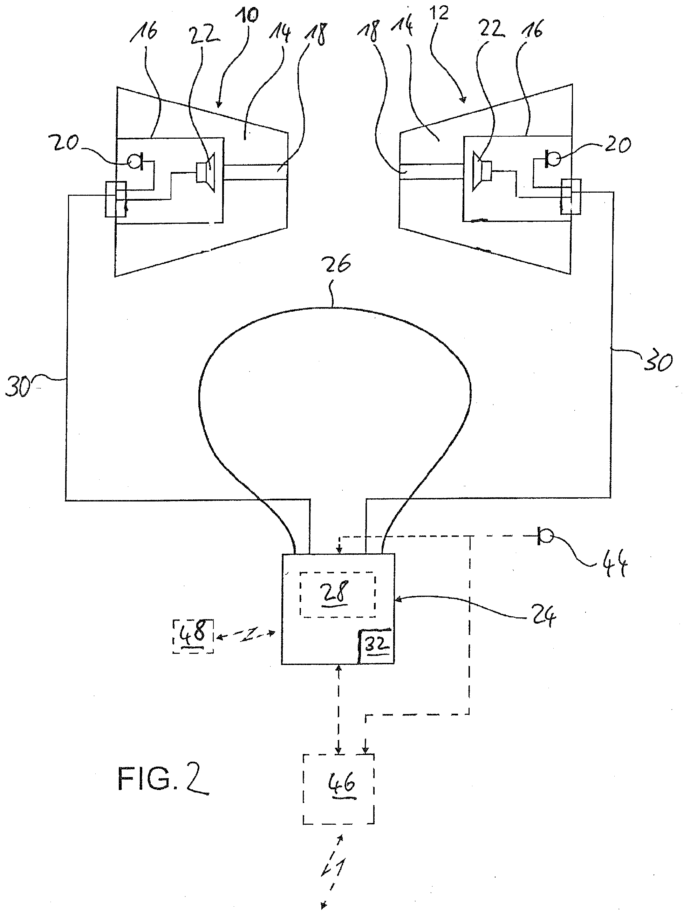

Fig. 2 is a schematic representation of a hearing protection system for a user comprising a hearing protection earplug 10 which is to be worn at least partly within the user's right ear canal and a hearing protection earplug 12 which is to be worn at least partly within the user's left ear canal. In a less preferred embodiment the earplugs 10, 12 may be replaced by hearing protection devices which are to be worn at the user's right and left ear, respectively, such as an earmuff.

Each hearing protection earplug 10, 12 comprises a shell 14 which is adapted to be worn at least in part in the user's ear canal, i.e. at least a distal portion of the shell could be inserted into the outer part of the user's ear canal in order to protect the user from excessive levels of ambient sound.

Preferably, the shell 14 is a customized shell, i.e. a hard or soft but firm shell having an outer surface individually shaped according to the inner shape of the user's outer ear and ear canal, which may be measured, for example, by direct laser scanning or by forming an impression. The customized shell may be produced by an additive process, such as layer-by-layer sintering of a powder material. Customized earplugs are described, for example, in US 2003/0133583 A1.

Typically, the shell 14 will provide for a (passive) acoustic attenuation of about 25 dB for medium frequencies (the attenuation is higher for higher frequencies, for example, increasing from about 20 dB at low frequencies to about 30 dB for high frequencies).

Each earplug 10, 12 is also provided with an active unit 16 for adjusting the frequency dependency of the attenuation and the degree of attenuation provided by each earplug 10, 12. The active unit 16 typically is inserted into a corresponding receptacle of the shell 14 and is locked there by corresponding locking means (not shown in Fig. 2) in a releasable manner. Thereby the shell 14 can be easily replaced, for example, if damaged.

The shell 14 is provided with a sound channel 18, by which the active unit 16 is acoustically connected to the ear canal. Preferably the shell 14 is designed such that it provides for an acoustic attenuation of at least 10 dB averaged over the audible frequency range when worn by the user.

The active unit 16 comprises a microphone 20 for capturing audio signals from ambient sound and a loudspeaker 22 for providing audio signals to the user's ear canal via the sound channel 18. Earplugs comprising an active unit are described, for example, in EP 1 674 059 A1.

The system also comprises a central unit 24 which is to be worn at the user's body below the user's neck, for example, by a loop 26 around the user's neck, and which comprises an audio signal processing unit 28 for receiving and processing the audio signals captured by the microphones 20, in order to supply the loudspeakers 22 with audio signals to be reproduced to the user's ear. To this end, the active units 16 are connected to the central unit 14 via cable connections 30 or wirelessly, such as via an inductive link (not shown). The central unit 24 may be provided with a user interface 32 comprising, for example, a button or a wheel for enabling the user to manually control the function of the audio signal processing unit 28.

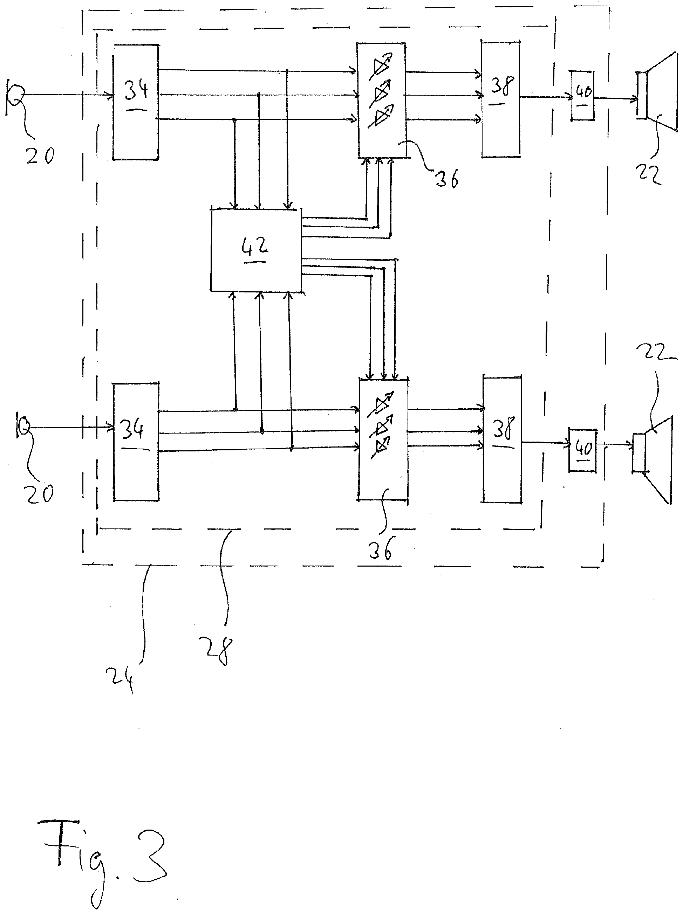

For each of the active units 16 the audio signal processing unit 28 comprises a unit 34 (see Fig. 3) for dividing the input audio signals provided by the microphone 20 into a plurality of frequency bands, a unit 36 for selective amplification of each of the frequency bands, i.e. a multi-channel variable gain amplifier, and a unit 38 for generating a single time domain filtered audio signal from the frequency channels, which filtered audio signal is supplied to an output driver 40 which drives the loudspeaker 22. The frequency divided input audio signals are also supplied to a control unit 42 which is designed such that it selects the gain applied within each frequency band in the amplifier unit 36 in such a manner that, by taking into account the shapes of the curves of equal loudness (see Fig. 1) and the passive acoustic attenuation provided by the shell 14 of the earplugs 10, 12, the total attenuation provided by each of the earplugs 10, 12 in each of the frequency bands corresponds to the same target loudness difference.

In other words, the gain model implemented in the audio signal processing unit 28 is such that the user experiences for all frequencies the same loudness reduction of the ambient sound, so that a hearing impression as close to natural as possible is achieved. For example, if the target loudness difference is 10 phons, an input level of 90 dB SPL at 1 kHz is damped by 10 dB and an input level of 115 dB SPL at 31.5 Hz is damped by 5 dB (according to Fig. 1, the vertical distance between two loudness curves differing by 10 phons is about 10 dB at 1 kHz and only 5 dB at low frequencies).

Preferably, the audio signal processing unit 28 is designed to select for each frequency band the same gain for both earplugs 10, 12, with the gain for each frequency band being selected according to the higher one of the sound pressure levels of the input audio signals captured by the left earplug 10 and right ear plug 12. Thereby localization cues based on level differences between the left and right ear can be preserved as far as possible.

Preferably, the audio signal processing unit 28 is designed to select the target loudness difference as a function of the sound pressure level of the input audio signals, in particular in such a manner that the target loudness difference increases with increasing sound pressure level of the input audio signals. For example, at low input levels, (e.g. up to about 75 dB) there may no attenuation at all (gain = 0), whereas at high input levels the attenuation may be, for example, 20 dB (gain = -20 dB) at 1 kHz, so that the attenuation for low input levels would be 0 phons, whereas for high levels it would be 20 phons (the maximum attainable attenuation, of course, is the passive attenuation provided by the shell 14). Preferably, the target loudness difference is selected according to the maximum SPL of all frequency bands of the input audio signals, i.e. the largest SPL occuring in any of the frequency bands. Alternatively or in addition the audio signal processing unit 28 may be designed in such a manner that the target loudness level is manually selectable by the user via the user interface 32, so that the user may individually adjust the attenuation to his personal preferences.

Preferably, the audio signal processing unit 28 is designed in such a manner that release times are between 100 and 200 ms and attack times are between 1 and 10 ms.

The skilled person will understand that the central unit 24 includes the necessary analog-to-digital and digital-to-analog converters and a battery (which are not shown). Sampling rates must be high enough to preserve also the high audible frequencies in the signal. This is important in view of the fact that the hearing protection systems of the present invention are primarily intended for being used by professional musicians. However, the system also may be used, for examples, for patients suffering from hyperacusis.

In addition to the components discussed so far, the hearing protection system could be provided with a communication function, wherein at least one additional microphone would be provided for capturing the user's voice. Such additional microphone may be a boom microphone attached to one of the earplugs (as indicated in Fig. 2 at 44 in dashed lines), or each of the earplugs may be provided with an additional microphone facing towards the ear canal, in order to pick up the user's voice by using a blind source separation algorithm (see, for example, WO 2007/073818 A1). An example of a system comprising a boom microphone is described in WO 2007/082579 A2. In both cases, the audio signal processing unit would be connected to a communication device, for example, an FM transceiver (as indicated in Fig. 2 at 46), in order to send the audio signals corresponding to the captured voice of the user to another person or to receive audio signals to be presented via the loudspeakers 22 from an external source, such as another person.

According to an alternative embodiment, the user interface 32 may be implemented as a remote control (indicated in Fig. 2 at 48) rather than being provided as part of the central unit 24.

Preferably, there will be at least 24 frequency bands / channels.

## Claims

1. A hearing protection system comprising a first hearing protection device (10) to be worn at a user's right outer ear and/or at least in part in the user's right ear canal and a second hearing protection device (12) to be worn at the user's left outer ear and/or at least in part in the user's left ear canal, each hearing protection device comprising an active unit (16) comprising at least one microphone (20) for converting ambient sound into input audio signals and a loudspeaker (22) for transforming filtered audio signals into sound perceivable by said user, the system further comprising an audio signal processing unit (28) for processing said input audio signals into said filtered audio signals, wherein the audio signal processing unit is designed to divide, for each of the hearing protection devices, the input audio signals into a plurality of frequency bands and to select the gain applied within each frequency band as a function of the frequency of the respective frequency band and the sound pressure level of the input audio signals falling within the respective frequency band in such a manner that, by taking into account the shapes of the curves of equal loudness and the passive attenuation provided by the respective hearing protection device, the total attenuation provided by the respective hearing protection device in each of the frequency bands corresponds to the same target loudness difference.

2. The system of claim 1, wherein the audio signal processing unit (28) is designed to select for each frequency band the same gain for both the first (10) and the second hearing protection device (12), and wherein the gain for each frequency band is selected according to the higher one of the sound pressure levels of the input audio signals captured by the first and second hearing protection device, respectively.

3. The system of one of claims 1 and 2, wherein the audio signal processing unit (28) is designed to select the target loudness difference as a function of the maximum of the sound pressure level of all frequency bands of the input audio signals.

4. The system of claim 3, wherein the audio signal processing unit (28) is designed to select the target loudness difference in a manner so as to increase with increasing maximum of the sound pressure level of all frequency bands of the input audio signals.

5. The system of one of claims 1 and 2, wherein the audio signal processing unit (28) is designed in such a manner that the target loudness level is manually selectable by the user via a user interface (32).

6. The system of one of the preceding claims, wherein the audio signal processing unit (28) is designed in such a manner that release times are between 100 and 200 ms.

7. The system of one of the preceding claims, wherein the audio signal processing unit (28) is designed in such a manner that attack times are between 1 and 10 ms.

8. The system of one of the preceding claims, wherein the first and second hearing protection devices are earplugs (10, 12).

9. The system of claim 8, wherein the audio signal processing unit (28) is contained in a housing (24) to be worn at the user's body below the user's neck, and wherein the active units (16) are connected to the audio signal processing unit (28) by cables (30) or wirelessly.

10. The system of one of claims 8 and 9, wherein each of the earplugs (10, 12) comprises a shell (14) into which the active unit (16) is inserted in a releasable manner.

11. A method for providing active hearing protection to a user, comprising:
providing a first hearing protection device (10) at the user's right outer ear and/or at least in part in the user's right ear canal and a second hearing protection device (12) at the user's left outer ear and/or at least in part in the user's left ear canal;
converting, in each hearing protection device, ambient sounds into input audio signals,
processing the input audio signals into filtered audio signals, for each of the hearing protection devices, by dividing the input audio signals into a plurality of frequency bands and selecting the gain applied within each frequency band as a function of the frequency of the respective frequency band and the sound pressure level of the input audio signals falling within the respective frequency band in such a manner that, by taking into account the shapes of the curves of equal loudness and the passive attenuation provided by the respective hearing protection device, the total attenuation provided by the respective hearing protection device in each of the frequency bands corresponds to the same target loudness difference, and
converting, in each hearing protection device, the filtered audio signals into sound perceivable by said person.

## Patentansprüche

1. Gehörschutzsystem mit einem ersten Gehörschutzgerät (10), welches am rechten Außenohr des Nutzers und/oder mindestens zum Teil im rechten Gehörgang des Nutzers zu tragen ist, und einem zweiten Gehörschutzgerät (12), welches am linken Außenohr des Nutzers und/oder mindestens zum Teil im linken Gehörgang des Nutzers zu tragen ist, wobei jedes Gehörschutzgerät eine aktive Einheit (16) aufweist, die mindestens ein Mikrofon (20) zum Umwandeln von Umgebungsschall in Eingangsaudiosignale und einen Lautsprecher (22) zum Umwandeln von gefilterten Audiosignalen in von dem Nutzer wahrnehmbaren Schall aufweist, wobei das System ferner eine Audiosignalverarbeitungseinheit (28) zum Verarbeiten der Eingangsaudiosignale in gefilterte Audiosignale aufweist, wobei die Audiosignalverarbeitungseinheit ausgebildet ist, um für jedes der Gehörschutzgeräte die Eingangsaudiosignale in eine Mehrzahl von Frequenzbändern zu unterteilen und die in jedem Frequenzband anzuwendende Verstärkung als Funktion der Frequenz des jeweiligen Frequenzbandes und des Schalldruckpegels der in das jeweilige Frequenzband fallenden Eingangsaudiosignalen so auszuwählen, dass unter Berücksichtigung der Formen der Kurven gleicher Lautheit und der von dem jeweiligen Gehörschutzgerät geleisteten passiven Dämpfung die von dem jeweiligen Gehörschutzgerät geleistete Gesamtdämpfung in jedem der Frequenzbänder der gleichen Ziellautheitsdifferenz entspricht.

2. System gemäß Anspruch 1, wobei die Audiosignalverarbeitungseinheit (28) ausgebildet ist, um für jedes Frequenzband für sowohl das erste Gehörschutzgerät (10) und das zweite Gehörschutzgerät (12) dieselbe Verstärkung auszuwählen, und wobei die Verstärkung für jedes Frequenzband entsprechend dem höheren Schalldruckpegel der Schalldruckpegel der von der ersten Gehörschutzvorrichtung bzw. der zweiten Gehörschutzvorrichtung aufgefangenen Audiosignale ausgewählt wird.

3. System gemäß einem der Ansprüche 1 und 2, wobei die Audiosignalverarbeitungseinheit (28) ausgelegt ist, um die Ziellautheitsdifferenz als Funktion des Maximums der Schalldruckpegel aller Frequenzbänder der Eingangsaudiosignale auszuwählen.

4. System gemäß Anspruch 3, wobei die Audiosignalverarbeitungseinheit (28) ausgelegt ist, um die Ziellautheitsdifferenz so auszuwählen, dass sie mit zunehmendem Maximum des Schalldruckpegels aller Frequenzbänder der Eingangsaudiosignale ansteigt.

5. System gemäß einem der Ansprüche 1 und 2, wobei die Audiosignalverarbeitungseinheit (28) in einer solchen Weise ausgelegt ist, dass der Ziellautheitspegel über eine Nutzerschnittstelle (32) manuell von dem Nutzer wählbar ist.

6. System gemäß einem der vorhergehenden Ansprüche, wobei die Audiosignalverarbeitungseinheit (28) so ausgelegt ist, dass die Abklingzeiten zwischen 100 und 200 ms liegen.

7. System gemäß einem der vorhergehenden Ansprüche, wobei die Audiosignalverarbeitungseinheit (28) so ausgelegt ist, dass die Ansprechzeiten zwischen 1 und 10 ms liegen.

8. System gemäß einem der vorhergehenden Ansprüche, wobei es sich bei dem ersten und dem zweiten Gehörschutzgerät um Ohrstopfen (10, 12) handelt.

9. System gemäß Anspruch 8, wobei die Audiosignalverarbeitungseinheit (28) in einem Gehäuse (24) enthalten ist, das am Körper des Nutzers unterhalb des Nackens des Nutzers zu tragen ist, und wobei die aktiven Einheiten (16) mittels Kabeln (30) oder drahtlos mit der Audiosignalverarbeitungseinheit (28) verbunden sind.

10. System gemäß einem der Ansprüche 8 und 9, wobei jeder der Ohrstopfen (10, 12) eine Schale (14) aufweist, in welche die aktive Einheit (16) in lösbarer Weise eingesetzt ist.

11. Verfahren zum Bereitstellen eines aktiven Gehörschutzes für einen Nutzer, wobei:
ein erstes Gehörschutzgerät (10) am rechten Außenohr des Nutzers und/oder mindestens zum Teil im rechten Gehörgang des Nutzers bereitgestellt wird und ein zweites Gehörschutzgerät (12) am linken Außenohr des Nutzers und/oder mindestens zum Teil im linken Gehörgang des Nutzers bereitgestellt wird;
in jedem der Gehörschutzgeräte Umgebungsschall in Eingangsaudiosignale umgewandelt wird;
die Eingangsaudiosignale für jedes der Gehörschutzgeräte in gefilterte Audiosignale verarbeitet werden, indem die Eingangsaudiosignale in eine Mehrzahl von Frequenzbändern aufgeteilt werden und die innerhalb jedem Frequenzband angewandte Verstärkung als Funktion der Frequenz des jeweiligen Frequenzbands und des Schalldruckpegels der in das jeweilige Frequenzband fallenden Audiosignale so ausgewählt wird, dass unter Berücksichtigung der Formen der Kurven gleicher Lautheit und der von dem jeweiligen Gehörschutzgerät geleisteten passiven Dämpfung die von dem jeweiligen Gehörschutzgerät geleistete Gesamtdämpfung in jedem der Frequenzbänder der gleichen Ziellautheitsdifferenz entspricht, und
in jedem Gehörschutzgerät die gefilterten Audiosignale in von dem Nutzer wahrnehmbaren Schall umgewandelt werden.

## Revendications

1. Système de protection auditive comprenant un premier dispositif de protection auditive (10) destiné à être porté au niveau de l'oreille externe droite d'un utilisateur et/ou au moins en partie dans le conduit auditif droit de l'utilisateur et un deuxième dispositif de protection auditive (12) destiné à être porté au niveau de l'oreille externe gauche de l'utilisateur et/ou au moins en partie dans le conduit auditif gauche de l'utilisateur, chaque dispositif de protection auditive comprenant une unité active (16) comprenant au moins un microphone (20) pour convertir un son ambiant en signaux audio d'entrée et un haut-parleur (22) pour transformer des signaux audio filtrés en un son perceptible par ledit utilisateur, le système comprenant en outre une unité de traitement de signaux audio (28) pour traiter lesdits signaux audio d'entrée en lesdits audio filtrés, l'unité de traitement de signaux audio étant conçue pour diviser, pour chacun des dispositifs de protection auditive, les signaux audio d'entrée en une pluralité de bandes de fréquences et pour sélectionner le gain appliqué dans chaque bande de fréquences en fonction de la fréquence de la bande de fréquences respective et du niveau de pression acoustique des signaux audio d'entrée tombant dans la bande de fréquences respective, de telle sorte que, tenant compte des formes des courbes de même intensité sonore et de l'atténuation passive fournie par le dispositif de protection auditive respectif, l'atténuation totale fournie par le dispositif de protection auditive respectif dans chacune des bandes de fréquences corresponde à la même différence d'intensité sonore cible.

2. Système selon la revendication 1, dans lequel l'unité de traitement de signaux audio (28) est conçue pour sélectionner, pour chaque bande de fréquences, le même gain pour le premier (10) et pour le deuxième (12) dispositif de protection auditive, et dans lequel le gain pour chaque bande de fréquences est sélectionné en fonction du plus élevé des niveaux de pression acoustique des signaux audio d'entrée capturés, respectivement, par les premier et deuxième dispositifs de protection auditive.

3. Système selon l'une des revendications 1 et 2, dans lequel l'unité de traitement de signaux audio (28) est conçue pour sélectionner la différence d'intensité sonore cible en fonction du maximum du niveau de pression acoustique de toutes les bandes de fréquences des signaux audio d'entrée.

4. Système selon la revendication 3, dans lequel l'unité de traitement de signaux audio (28) est conçue pour sélectionner la différence d'intensité sonore cible de manière à augmenter avec l'augmentation du maximum du niveau de pression acoustique de toutes les bandes de fréquences des signaux audio d'entrée.

5. Système selon l'une des revendications 1 et 2, dans lequel l'unité de traitement de signaux audio (28) est conçue de telle sorte que le niveau sonore cible puisse être sélectionné manuellement par l'utilisateur via une interface utilisateur (32).

6. Système selon l'une des revendications précédentes, dans lequel l'unité de traitement de signaux audio (28) est conçue de telle sorte que les temps de relâchement soient compris entre 100 et 200 ms.

7. Système selon l'une des revendications précédentes, dans lequel l'unité de traitement de signaux audio (28) est conçue de telle sorte que les temps d'attaque soient compris entre 1 et 10 ms.

8. Système selon l'une des revendications précédentes, dans lequel les premier et deuxième dispositifs de protection auditive sont des bouchons d'oreille (10, 12).

9. Système selon la revendication 8, dans lequel l'unité de traitement de signaux audio (28) est contenue dans un boîtier (24) destiné à être porté au niveau du corps de l'utilisateur, en dessous du cou de l'utilisateur, et dans lequel les unités actives (16) sont connectées à l'unité de traitement de signaux audio (28) par des câbles (30) ou par une connexion sans fil.

10. Système selon l'une des revendications 8 et 9, dans lequel chacun des bouchons d'oreille (10, 12) comprend une coque (14) dans laquelle l'unité active (16) est insérée d'une manière libérable.

11. Procédé permettant de fournir une protection auditive active à un utilisateur, comprenant :
la prévision d'un premier dispositif de protection auditive (10) au niveau de l'oreille externe droite de l'utilisateur et/ou au moins en partie dans le conduit auditif droit de l'utilisateur et d'un deuxième dispositif de protection auditive (12) au niveau de l'oreille externe gauche de l'utilisateur et/ou au moins en partie dans le conduit auditif gauche de l'utilisateur ;
la conversion, dans chaque dispositif de protection auditive, de sons ambiants en signaux audio d'entrée ;
le traitement des signaux audio d'entrée en lesdits signaux audio filtrés, pour chacun des dispositifs de protection auditive, par division des signaux audio d'entrée en une pluralité de bandes de fréquences et sélection du gain appliqué dans chaque bande de fréquences en fonction de la fréquence de la bande de fréquences respective et du niveau de pression acoustique des signaux audio d'entrée tombant dans la bande de fréquences respective, de telle sorte que, tenant compte des formes des courbes de même intensité sonore et de l'atténuation passive fournie par le dispositif de protection auditive respectif, l'atténuation totale fournie par le dispositif de protection auditive respectif dans chacune des bandes de fréquences corresponde à la même différence d'intensité sonore cible, et
la conversion, dans chaque dispositif de protection auditive, des signaux audio filtrés en un son perceptible par ladite personne.
